# EUROPEAN PATENT SPECIFICATION

(11) **EP 1 245 570 B1**
(45) Date of publication and mention of the grant of the patent: **29.12.2004**
(21) Application number: 01303077.0
(22) Date of filing: 30.03.2001
(51) Int. Cl.: C07D 307/08

(54) **Process for the production of (-) 3,4 divanillyl tetrahydrofuran**
Verfahren zur Herstellung von (-) 3,4 Divanillyltetrahydrofuran
Procédé pour la préparation de (-) 3,4 divanillyl tétrahydofurane

(43) Date of publication of application: 02.10.2002
(73) Proprietor: Council of Scientific and Industrial Research, New Delhi 110 001 (IN)
(72) Inventor: Chattopadhyay, Sunil Kumar, Lucknow 226015 Uttar Pradesh (IN); Srivastava, Sachin, Lucknow 226015 Uttar Pradesh (IN); Tripathi, Vinayak, Lucknow 226015 Uttar Pradesh (IN)
(74) Representative: Towler, Philip Dean

(56) References cited:
- SCHOETTNER M ET AL: "LIGNANS FROM THE ROOTS OF URTICA DIOICA AND THEIR METABOLITES BIND TO HUMAN SEX HORMONE BINDING GLOBULIN (SHBG)" PLANTA MEDICA, THIEME, STUTTGART, DE, vol. 63, 1997, pages 529-532, XP001012826 ISSN: 0032-0943
- MUJUMDAR R B ET AL: "TAXIRESINOL, A NEW LIGNAN IN THE HEARTWOOD OF TAXUS BACCATA" INDIAN JOURNAL OF CHEMISTRY, JODHPUR, IN, vol. 10, July 1972 (1972-07), pages 677-680, XP001012828
- BARRY C N ET AL: "TRIPHENYLPHOSPHINE-TETRACHLOROMETHANE PROMOTED CHLORINATION AND CYCLODEHYDRATION OF SIMPLE DIOLS" JOURNAL OF ORGANIC CHEMISTRY, AMERICAN CHEMICAL SOCIETY. EASTON, US, vol. 46, no. 16, 31 July 1981 (1981-07-31), pages 3361-3364, XP000673385 ISSN: 0022-3263
- SHU-FANG ET AL.: "Lycorine Alkaloids from Hymenocallis Littoralis" PHYTOCHEMISTRY, vol. 40, no. 4, 1995, pages 1295-1298, XP002177724

## Description

This invention relates to a process for the production of (-) 3,4-divanillyl tetrahydrofuran. More particularly, this invention relates to an improved process for the isolation of (-) secoisolariciresinol from the heart wood and roots of Taxus wallichiana and conversion of (-) secoisolariciresinol of formula (1) into its semisynthetic analog (-) 3,4-divanillyl tetrahydrofuran of formula (2).

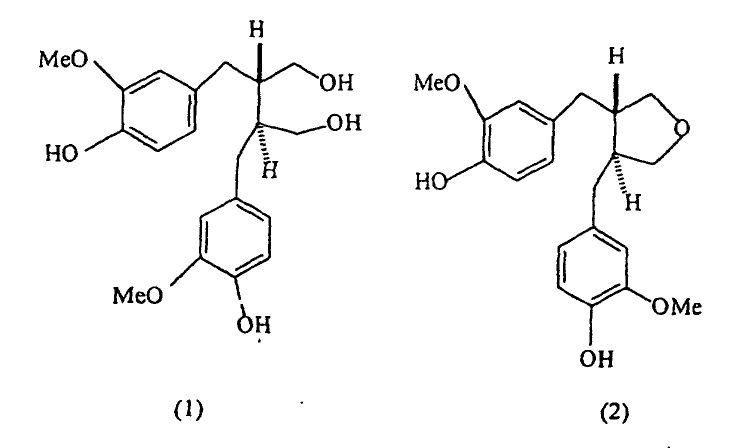

### Back ground of the invention

(-) secoisolariciresinol is a known molecule and it has previously been isolated from the heartwood of *T*. *wallichiana* but it has never been isolated from the roots of *T*. *wallichiana*. According to a prior art process, (-) secoisolariciresinol has been isolated from the heartwood of *T. wallichiana* by extracting the powdered heartwood with cold methanol and concentrating the methanol to get methanol extract, treating the methanol extract with hot petroleum-ether to remove waxes, and subjecting the dewaxed material to counter current distribution process between equal volumes of benzene, ethyl acetate, water and methanol, yielding three main fractions, subjecting the desired fraction containing (-) secoisolariciresinol to column chromatography and isolating (-) secoisolariciresinol from the column by eluting with benzene acetone as eluent with a yield of 0 1% (Taxiresinol, a new lignan in the heartwood of *Taxus haccata* [R B Mujumdar, R.Srinivasan and K. Venkataraman, Indian J. Chem 10, 677-680 (1972)].

The process described above for the isolation of (-) secoisolariciresinol from *T*. *wallichiana* suffers from several disadvantages which include, an extra step of defatting with petroleum ether, use of benzene (which is carcinogenic) as a solvent both in the counter current distribution process and also as a eluent during isolating from the column and the use of column chromatography which makes the process tedious, time consuming and require extra volumes of solvents. Schöttner et al., Planta Medica 63 (1997) 529-532 discloses the extraction of (-)-secoisolariciresinol from Urtica dioica. (-)-secoisolariciresinol so obtained is transformed into (-)-3,4-divanillyltetrahydrofurane by treatment with H₂SO₄/trimethyl orthoformate in MeOH.

According to another prior art process, the preparation of (-) 3,4-divanillyl tetrahydrofuran of formula (2) from (-) secoisolariciresinol of formula (1) involves the treatment of(-) secoisolariciresinol in an acetone solution with perchloric acid. The drawback of the process includes that the epoxide of the compound of formula (2) thus formed is unstable in acidic solution and it gets partly decomposed to the starting material i.e. (-) secoisolariciresinol of formula (1).

The plant *Taxus wallichiana* is an important plant from the medicinal point of view as it contains the anticancer compound paclitaxel (=taxol ^{(R)}). Paclitaxel, a highly oxygenated diterpenoid molecule and a potent anticancer drug for the treatments of ovarian and breast cancers, was first isolated from *Taxus brevifolia*. Thereafter, it has also been isolated from the Himalayan yew *Taxus wallichiana*. We have been working on different parts - stem bark, needles, heartwood, roots and seeds of *T. wallichiana* as a part of our systematic investigation of the plant for the isolation of paclitaxel, its important analogs, precursors and other biologically active molecules. During the course of this investigation, we have been able to isolate an important molecule (-) secoisolariciresinol of formula (1) from the heartwood as well as roots of this plant with high yields.

Recent findings have established that (-) secoisolariciresinol possesses significant aromatase inhibition property. Aromatase is a key enzyme in steroid hormone metabolism. It mediates the conversion of androgens into estrogens. Aromatase is of considerable therapeutic interest since estrogen production has been associated with endometrial and breast cancers. Aromatase inhibitors are used in treating estrogen dependent breast cancers.

(-) secoisolariciresinol is also capable of reducing the binding of ³H-labelled 5à-dihydrotestosterone (DHT) to human sex hormone binding globulin (SHBG) and thereby reduces the chance of growth of prostate cancer. However, the semisynthetic analog (-) 3,4-divanillyl tetrahydrofuran prepared from (-) secoisolariciresinol by the process of the invention is more active in reducing the binding of ³H-labelled 5à-dihydro testosterone (DHT) to human sex hormone binding globulin (SHBG) Thus (-) 3,4-divanillyl tetrahydrofuran is more active than (-) secoisolariciresinol in reducing the chance of growth of prostate cancers in humans.

### Objects of the invention

The object of the present invention is to develop an improved process for isolation of (-) secoisolariciresinol from the heartwood and roots of *T. wallichiana* with high yields.

Another object of the present invention is to develop a processing technology for the isolation of (-) secoisolariciresinol from the heartwood and roots of *T*. *wallichiana* which does not use any chromatographic separation for its isolation.

Yet another object of the present invention is to avoid the use of carcinogenic solvent benzene for isolation of (-) secoisolariciresinol.

Still another object of the present invention is to convert (-)secoisolariciresinol of formula (1) into its more active semisynthetic analog (-) 3,4-divanillyl tetrahydrofuran of formula (2) under neutral conditions.

Yet another object of the present invention is to convert (-) secoisolariciresinol into (-) 3,4-divanillyl tetrahydrofuran with high yields.

### Summary of the invention

In order to overcome the drawbacks of the prior art processes, we have developed a simple and practical process for production of (-) 3,4,-divanillyl tetrahydrofuran of formula (2) 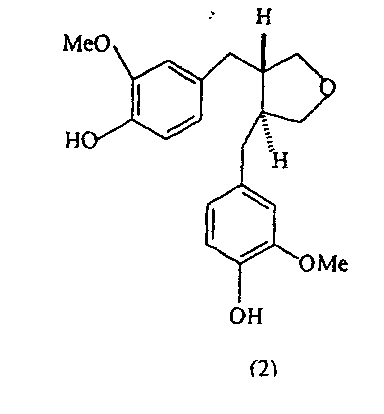 comprising (a) extracting the pulverized heartwood/roots of *T*. *wallichiana* with alcohol at ambient temperature and concentrating the solvent to give an alcoholic extract, (b) treating the alcoholic extract with water and extracting with a chlorinated solvent exhaustively (c) concentrating the chlorinated solvent to a residue and treating the residue with aqueous solution of a base and extracting the alkaline layer with an organic solvent (d) neutralizing the aqueous alkaline solution with mineral acid and extracting with an organic solvent (e) concentrating the organic solvent to a residue and crystallizing it from a suitable organic solvent or mixture of such solvents to get (-) secoisolariciresinol of formula (1), 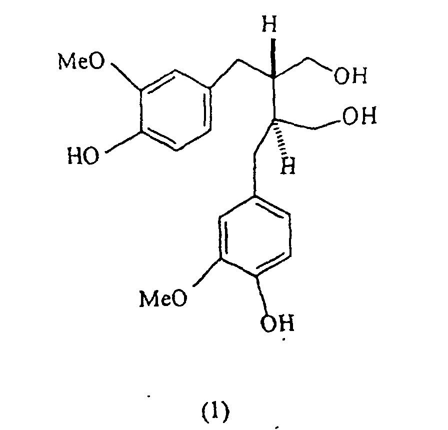 (f) dissolving the isolated (-) secoisolariciresinol in an organic solvent and reacting it with triphenyl phosphine halide at 0-80°C for 1-10 hours and (g) isolating (-) 3,4-divanillyl tetrahydrofuran of formula (2) by column chromatography.

Accordingly the present invention relates to a process for the preparation of (-) 3,4-divanillyl tetrahydrofuran of formula (2), said process comprising (f) dissolving the isolated (-) secoisolariciresinol in an organic solvent, reacting it with triphenyl phosphine halide at 0-80°C for 1-10 hours and (g) isolating (-) 3,4-divanillyl tetrahydrofuran of formula (2) by column chromatography.

In one embodiment of the present invention the alcohol used in step (a) is an alkanol selected from the group consisting of methanol and ethanol.

In another embodiment of the invention, the chlorinated solvent in step (b) is selected from the group consisting of chloroform and dichloromethane

In yet another embodiment of the invention, the base used in step (c) is selected from the group consisting of sodium hydroxide, potassium hydroxide and lithium hydroxide.

In a further embodiment of the present invention, the organic solvent used in step (c) is selected from the group consisting of toluene, chloroform, dichloromethane and ethyl acetate.

In yet another embodiment of the present invention, the mineral acid in step (d) is selected from the group consisting of hydrochloric acid and sulphuric acid.

In yet another embodiment of the invention, the suitable organic solvent or mixtures of such solvents used in step (e) is selected from the group consisting of acetone, and mixtures of acetone-petroleum ether, acetone-hexane, ethyl acetate-pet ether and ethylacetate-hexane.

In another embodiment of the invention, the organic solvent used in step (f) for dissolving (-) secoisolariciresinol, is selected from the group consisting of acetonitrile, methanol, tetrahydrofuran, chloroform and dichloromethane.

In a yet another embodiment of the present invention, the triphenyl phosphine halide used in step (f) is selected from a group consisting of triphenyl phosphine bromide and triphenylphosphine chloride.

In a further embodiment of the present invention, the colum used for chromatography in step (g) is selected from a group consisting of silica gel, florosil, alumina and celite.

### Detailed description of the invention

The process of the invention for the isolation of (-) secoisolariciresinol from the heartwood and roots of *T. wallichiana* does not use column chromatography. The improved isolation process comprises (a) extracting the pulverized heartwoods/roots of *T. wallichiana* with an alcohol at ambient temperature and concentrating the solvent to obtain an alcoholic extract, (b) treating the alcoholic extract with water and extracting it with chlorinated solvents exhaustively (c) concentrating the chlorinated solvent to a residue and treating the resultant residue with aqueous solution of a base and extracting the alkaline layer with an organic solvent (d) neutralizing the alkaline solution with mineral acid and extracting with an organic solvent, (e) concentrating the organic solvent to a residue and crystallizing it from a suitable organic solvent or mixtures of such solvents to get (-) secoisolariciresinol of formula (1).

The isolated (-) secoisolariciresinol of formula (1) is converted into (-) 3,4-divanillyl tetrahydrofuran of formula (2) by (f) dissolving the isolated (-) secoisolariciresinol in an organic solvent, reacting it with triphenyl phosphine halide at 0-80°C for 1-10 hours and (g) isolating (-) 3,4-divanillyl tetrahydrofuran of formula (2) by column chromatography.

The alcohol used in step (a) can be an alkanol such as methanol and ethanol. The chlorinated solvent in step (b) is selected from the group consisting of chloroform and dichloromethane. The base used in step (c) is selected from the group consisting of sodium hydroxide, potassium hydroxide and lithium hydroxide.

The organic solvent used in step (c) is selected from the group consisting of toluene, chloroform, dichloromethane, ethyl acetate. The mineral acid in step (d) is selected from the group consisting of hydrochloric acid, sulphuric acid. The suitable organic solvent or mixtures of such solvents used in step (e) comprises acetone, and mixtures of acetone-petroleum ether, acetone-hexane, ethyl acetate-pet ether, ethylacetate-hexane or any mixture thereof.

The organic solvent used in step (f) for dissolving (-) secoisolariciresinol, is selected from the group consisting of acetonitrile, methanol, tetrahydrofuran, chloroform, dichloromethane. The triphenyl phosphine halide used in step (f) is selected from a group consisting of triphenyl phosphine bromide, triphenylphosphine chloride.

The column used for chromatography in step (g) is selected from a group consisting of silica gel, florosil, alumina, celite

The invention is described in detail in the examples given below which are provided to illustrate the invention and therefore should not be construed to limit the scope of the invention.

### Example I

### Production of(-) secoisolariciresinol from heartwood:

Air dried pulverized heartwood (1kg) of *T. wallichiana* was extracted with MeOH (5 lit x 3) at ambient temperature for three days. The MeOH extract was concentrated to a residue and the residue was treated with water (2 litres) and extracted with CHCl₃ (3 lit x 3) exhaustively. The CHCl₃ was concentrated under vacuum and the residue thus obtained was dissolved in 1N NaOH solution (1 lit) with stirring and extracted with CHCl₃ (1 lit x 3). The aqueous NaOH layer was neutralized with 1N HCl and extracted with ethylacetate (1 lit x 3). The ethyl acetate layer was washed with water, dried over anhydrous sodium sulphate and concentrated to a residue which crystallized out and filtered. It was recrystallized from a mixture of acetone-pet. ether to give (-) secoisolariciresinol (2g) mp. 112-113°C, [α]_{D}-25° (Cl, MeOH).

### Example II

### Production of (-) secoisolariciresinol from roots:

Air dried, pulverized roots (1kg) were extracted and processed as per process given in example I to give (-) secoisolariciresinol m.p. 112-113°C, [α]_{D}-25° (Cl, MeOH), yield 2.01g.

### Example III

### Production of (-) secoisolariciresinol from heartwood:

Air dried pulverized heartwood (1kg) of *T. wallichiana* were extracted with EtOH (5 lit x 3) at ambient temperature for three days. The EtOH extract was concentrated to a residue and the residue was treated with water (2 litre) and extracted with CH₂Cl₂ (3 lit x 3) exhaustively. The CH₂Cl₂ extract was concentrated under vacuum and the residue thus obtained was dissolved in 1N KOH solution (1 lit) with stirring and extracted with CH₂Cl₂ (1 lit x 3). The aqueous KOH layer was neutralized with 1N H₂SO₄ and extracted with CHCl₃, (2 lit x 3). The CHCl₃ layer was washed with water, dried over anhydrous sodium sulphate and concentrated to give a residue which crystallized out. The crystals were filtered and recrystallized from a mixture of acetone-hexane to give (-) secoisolariciresinol (2g) mp 112-113°C, [α]_{D}-25° (Cl, MeOH).

### Example IV

### Production of (-) secoisolariciresinol from roots:

Air dried, pulverized roots (1 kg) were extracted and processed as per process given in example III to give (-) secoisolariciresinol, mp. 112-113°C, [α]_{D}-25° (Cl, MeOH), yield (2.01g).

### Example V

### Production of (-) 3,4-divanillyl tetrahydrofuran

(-) secoisolariciresinol (100mg) was dissolved in acetonitrile (5ml) a solution of triphenyl phosphine bromide in acetonitrile was added, (1 ml) and the mixture was stirred at 0-80°C for 1-10 hours. After completion of the reaction, the reaction mixture was concentrated under vacuum and the residue thus obtained was chromatographed over a silica gel column. Elution of the column with 30% EtOAC in pet. ether gave (-) 3,4-divanillyl tetrahydrofuran (78mg), m.p. 114-116°C.

### Example VI

### Production of (-) 3,4-divanillyl tetrahydrofuran

(-) secoisolariciresinol (100mg) was dissolved in THF (5ml) a solution of triphenyl phosphine chloride in acetonitrile (1 ml) was added and the mixture was stirred at 0-80°C for 1-10 hours. After completion of the reaction, the reaction mixture was concentrated under vacuum and the residue thus obtained was chromatographed over a florosil column. Elution of the column with 30% EtOAC in pet. ether gave (-) 3,4-divanillyl tetrahydrofuran (77mg), m.p. 114-116°C.

### Advantages

1. The process developed for isolation of (-) secoisolariciresinol from the heartwood/roots of *T. wallichiana* is simple, practical and straightforward. No extreme conditions are necessary for its isolation.
2. The process developed for isolation of (-) secoisolariciresinol did not need any - chromatographic separation. Thus, the process is cost effective and viable for commercial production.
3. The solvent used for extraction can be recycled and thus the process is cost effective.
4. Compared to prior art process, (-) 3,4-divanillyl tetrahydrofuran has been prepared from (-) secoisolariciresinol under neutral conditions, and the product is obtained in excellent yield (90%).
5. The yields of (-) secoisolariciresinol and (-) 3,4-divanillyl tetrahydrofuran obtained by the process of the present invention have been compared with the corresponding yields reported earlier.

| Source | (-) secoiso-lariciresinol | (-) 3,4 divanillyl tetrahydrofuran |
|---|---|---|
| *Taxus wallichiana* | 0.1% | - |
| (heartwood) | | |
| Previous art | - | No yield reported |
| | | (However, 40% yield |
| | | obtained following |
| | | the prior art process) |
| Present process | 0.2% | >90% |

## Claims

1. A process for production of (-)3,4-divanillyl tetrahydrofuran of formula (2) (2) 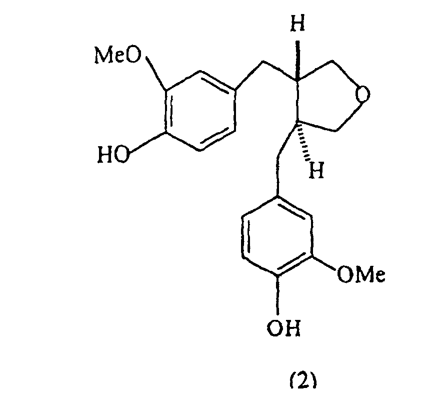 said process comprising (a) extracting the pulverized heartwoods/roots of *T. wallichiana* with an alcohol at ambient temperature and concentrating the solvent to obtain an alcoholic extract, (b) treating the alcoholic extract with water and extracting it with a chlorinated solvent exhaustively (c) concentrating the chlorinated solvent to a residue and treating the resultant residue with aqueous solution of a base and extracting the alkaline layer with an organic solvent (d) neutralizing the alkaline solution with mineral acid and extracting with an organic solvent, (e) concentrating the organic solvent to a residue and crystallizing it from a suitable organic solvent or mixtures of such solvents to get (-) secoisolariciresinol of formula (1). 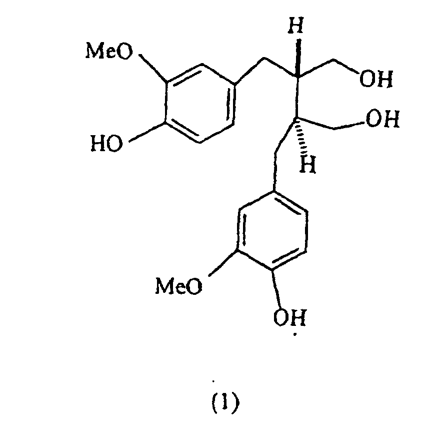 (f) dissolving the isolated (-) secoisolariciresinol in an organic solvent, reacting it with triphenyl phosphine halide at 0-80°C for 1-10 hours and (g) isolating (-) 3,4-divanillyl tetrahydrofuran of formula (2) by column chromatography.

2. A process as claimed in claim 1 wherein the alcohol used in step (a) is an alkanol selected from the group consisting of methanol and ethanol.

3. A process as claimed in claim 2 wherein the alcohol is methanol.

4. A process as claimed in any one of claims 1 to 3 wherein the chlorinated solvent used in step (b) is selected from chloroform and dichloromethane.

5. A process as claimed in claim 4 wherein the chlorinated solvent is chloroform

6. A process as claimed in any one of claims 1 to 5 wherein the base used in step (c) is selected from the group consisting of sodium hydroxide, potassium hydroxide and lithium hydroxide.

7. A process as claimed in claim 6 wherein the base is sodium hydroxide.

8. A process as claimed in any one of claims 1 to 7 wherein the organic solvent used to extract the alkaline layer in step (c) is selected from the group consisting of toluene, chloroform, dichloromethane and ethyl acetate.

9. A process as claimed in claim 8 wherein the organic solvent is chloroform.

10. A process as claimed in any one of claims 1 to 9 wherein the mineral acid used in step (d) to neutralize the alkaline solution is selected from hydrochloric acid and sulphuric acid.

11. A process as claimed in claim 10 wherein the mineral acid is hydrochloric acid.

12. A process as claimed in any one of claims 1 to 11 wherein the organic solvent used in step (d) to extract the neutral solution is selected from the group consisting ethylacetate, chloroform and dichloromethane.

13. A process as claimed in claim 12 wherein the organic solvent is ethylacetate.

14. A process as claimed in any one of claims 1 to 13 wherein the suitable organic solvent or mixtures of such solvent used in step (e) to crystallize (-) secoisolariciresinol is selected from the group consisting of acetone, and mixtures of acetone-petroleum ether, acetone-hexane, ethyl acetate-pet. Ether and ethylacetate-hexane.

15. A process as claimed in claim 14 wherein the mixture of solvent is acetone petroleum ether mixture.

16. A process as claimed in any one of claims 1 to 15 wherein the organic solvent used in step (f) for dissolving (-) secoisolariciresinol, is selected from the group consisting of acetonitrile, methanol, tetrahydrofuran, chloroform and dichloromethane.

17. A process as claimed in claim 16 wherein the solvent is acetonitrile.

18. A process as claimed in any one of claims 1 to 17 wherein the triphenyl phosphine halide used in step (f) is selected from triphenyl phosphine bromide and triphenylphosphine chloride.

19. A process as claimed in claim 18 wherein the triphenyl phosphine halide is triphenyl phosphine bromide.

20. A process as claimed in any one of claims 1 to 19 wherein the reaction temperature of step (f) is 60°C and the time period of the reaction in step (f) is 2 hours.

21. A process as claimed in any one of claims 1 to 20 wherein the column used for chromatography in step (g) to purify (-) 3,4-divanillyl tetrahydrofuran is selected from the group consisting of silica gel, florosil, alumina and celite

22. A process as claimed in claim 21 wherein the column used for chromatography comprises silica gel.

## Patentansprüche

1. Verfahren zur Herstellung von (-)3,4-Divanillyltetrahydrofuran der Formel (2) 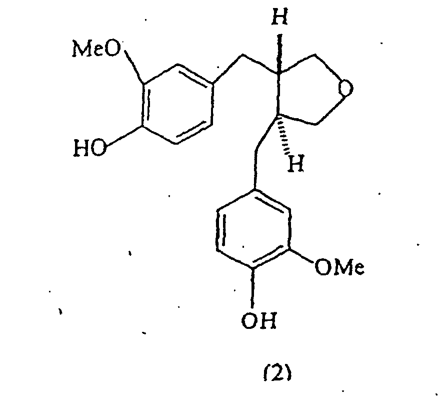 wobei das Verfahren umfasst (a) Extraktion der pulverisierten Kernhölzer/Wurzeln von *T. wallichiana* mit einem Alkohol bei Raumtemperatur und Konzentration des Lösungsmittels, um einen alkoholischen Extrakt zu erhalten, (b) Behandlung des alkoholischen Extrakts mit Wasser und dessen vollständige Extraktion mit einem chlorierten Lösungsmittel, (c) Konzentration des chlorierten Lösungsmittels auf eine Rest und Behandlung des resultierenden Restes mit einer wässrigen Lösung einer Base und Extraktion der alkalischen Schicht mit einem organischen Lösungsmittel, (d) Neutralisation der alkalischen Lösung mit Mineralsäure und Extraktion mit einem organischen Lösungsmittel, (e) Konzentration des organischen Lösungsmittels auf einen Rest und dessen Kristallisation aus einem geeigneten organischen Lösungsmittel oder Gemischen von solchen Lösungsmitteln, um (-) Secoisolariciresinol der Formel (1) zu erhalten 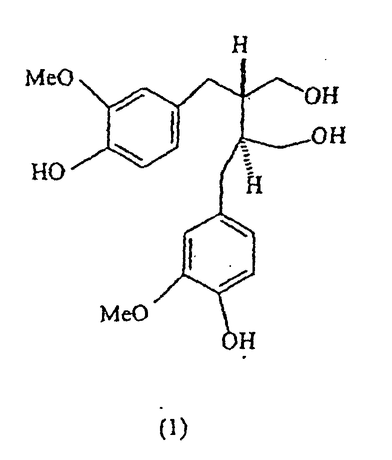 (f) Lösen des isolierten (-)Secoisolariciresinol in einem organischen Lösungsmittel, dessen Umsetzung mit Triphenylphosphinhalogenid bei 0-80 °C 1-10 Stunden lang und (g) Isolierung von (-)3,4-Divanillyltetrahydrofuran der Formel (2) mittels Säulenchromatographie.

2. Verfahren nach Anspruch 1, wobei der Alkohol, der in Schritt (a) verwendet wurde, ein Alkanol ausgewählt aus der Gruppe bestehend aus Methanol und Ethanol ist.

3. Verfahren nach Anspruch 2, wobei der Alkohol Methanol ist.

4. Verfahren nach einem der Ansprüche 1 bis 3, wobei das chlorierte Lösungsmittel, welches in Schritt (b) verwendet wird, ausgewählt ist aus Chloroform und Dichlormethan.

5. Verfahren nach Anspruch 4, wobei das chlorierte Lösungsmittel Chloroform ist.

6. Verfahren nach einem der Ansprüche 1 bis 5, wobei die Base, die Schritt (c) verwendet wird, ausgewählt ist aus der Gruppe bestehend aus Natriumhydroxid, Kaliumhydroxid und Lithiumhydroxid.

7. Verfahren nach Anspruch 6, wobei die Base Natriumhydroxid ist.

8. Verfahren nach einem der Ansprüche 1 bis 7, wobei das organische Lösungsmittel, das zur Extraktion der alkalischen Schicht in Schritt (c) verwendet ist, ausgesucht ist aus der Gruppe bestehend aus Toluol, Chloroform, Dichlormethan und Ethylacetat.

9. Verfahren nach Anspruch 8, wobei das organische Lösungsmittel Chloroform ist.

10. Verfahren nach einem der Ansprüche 1 bis 9, wobei die Mineralsäure, die in Schritt (d) verwendet wird, um die alkalische Lösung zu neutralisieren, ausgewählt ist aus Salzsäure und Schwefelsäure.

11. Verfahren nach Anspruch 10, wobei das Mineral Salzsäure ist.

12. Verfahren nach einem der Ansprüche 1 bis 11, wobei das organische Lösungsmittel, das in Schritt (d) verwendet wird, um die neutrale Lösung zu extrahieren, ausgewählt ist aus der Gruppe bestehend aus Ethylacetat, Chloroform und Dichlormethan.

13. Verfahren nach Anspruch 12, wobei das organische Lösungsmittel Ethylacetat ist.

14. Verfahren nach einem der Ansprüche 1 bis 13, wobei das geeignete organische Lösungsmittel oder Gemische solcher Lösungsmittel, die Schritt (e) verwendet werden, um (-)Secoisolariciresinol zu kristallisieren, ausgewählt sind aus der Gruppe bestehend aus Aceton und Gemischen Aceton-Petrolether, Acetonhexan, Ethylacetat-pet. Ether und Ethylacetat-Hexan.

15. Verfahren nach Anspruch 14, wobei das Lösungsmittelgemisch Aceton-Petrolether-Gemisch ist.

16. Verfahren nach einem der Ansprüche 1 bis 15, wobei das organische Lösungsmittel, das in Schritt (f) zur Auflösung von (-)Secoisolariciresinol verwendet wird, ausgewählt ist aus der Gruppe bestehend aus Acetonitril, Methanol, Tetrahydrofuran, Chloroform und Dichlormethan.

17. Verfahren nach Anspruch 16, wobei das Lösungsmittel Acetonitril ist.

18. Verfahren nach einem der Ansprüche 1 bis 17, wobei das Triphenylphosphinhalogenid, das in Schritt (f) verwendet wird, ausgewählt ist aus Triphenylphosphinbromid und Triphenylphosphinchlorid.

19. Verfahren nach Anspruch 18, wobei das Triphenylphosphinhalogenid triphenylphosphinbromid ist.

20. Verfahren nach einem der Ansprüche 1 bis 19, wobei die Reaktionstemperatur von Schritt (f) 60 °C ist und die Zeitdauer der Reaktion in Schritt (f) 2 Stunden beträgt.

21. Verfahren nach einem der Ansprüche 1 bis 20, wobei die Säule, die zur Chromatographie in Schritt (g) verwendet wird, um (-)3,4-Divanillyltetrahydrofuran aufzureinigen, ausgewählt ist aus der Gruppe bestehend aus Silicagel, Florosil, Aluminiumoxid und Celiten.

22. Verfahren nach Anspruch 21, wobei die Säule, die zur Chromatographie verwendet wird, Silicagel umfasst.

## Revendications

1. Procédé pour la production du (-)3,4-divanillyle tétrahydrofurane de formule (2). 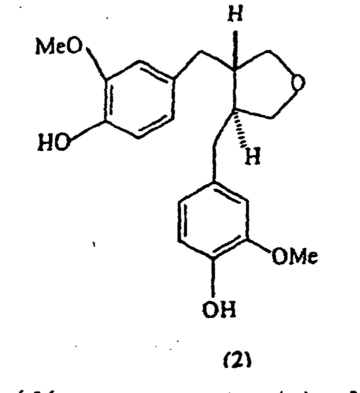 ledit procédé comprenant (a) l'extraction des duramens/racines pulvérisés de *T. wallichiana* avec un alcool à température ambiante et la concentration du solvant pour obtenir un extrait alcoolique, (b) le traitement de l'extrait alcoolique avec de l'eau et son extraction avec un solvant chloré de manière exhaustive, (c) la concentration du solvant chloré en un résidu et le traitement du résidu résultant avec une solution aqueuse d'une base et l'extraction de la couche alcaline avec un solvant organique, (d) la neutralisation de la solution alcaline avec un acide minéral et l'extraction avec un solvant organique, (e) la concentration du solvant organique en un résidu et sa cristallisation à partir d'un. solvant organique approprié ou de mélanges de tels solvants pour obtenir du (-)secoisolaricirésinol de formule (1). 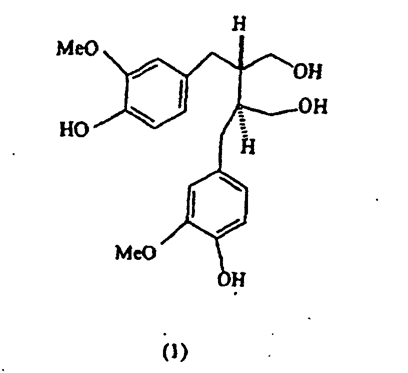 (f) la dissolution du (-)secoisolaricirésinol isolé dans un solvant organique, la réaction de celui-ci avec un halogénure de triphénylphosphine de 0 à 80°C pendant 1 à 10 heures et (g) l'isolation du (-)3,4-divanillyle tétrahydrofurane de formule (2) par chromatographie sur colonne.

2. Procédé tel que revendiqué dans la revendication 1 dans lequel l'alcool utilisé dans l'étape (a) est un alcanol choisi parmi le méthanol et l'éthanol.

3. Procédé tel que revendiqué dans la revendication 2 dans lequel l'alcool est du méthanol.

4. Procédé tel que revendiqué dans l'une quelconque des revendications 1 à 3 dans lequel le solvant chloré utilisé dans l'étape (b) est choisi parmi le chloroforme et le dichlorométhane.

5. Procédé tel que revendiqué dans la revendication 4 dans lequel le solvant chloré est du chloroforme.

6. Procédé tel que revendiqué dans l'une quelconque des revendications 1 à 5 dans lequel la base utilisée dans l'étape (c) est choisie parmi l'hydroxyde de sodium, l'hydroxyde de potassium et l'hydroxyde de lithium.

7. Procédé tel que revendiqué dans la revendication 6 dans lequel la base est de l'hydroxyde de sodium.

8. Procédé tel que revendiqué dans l'une quelconque des revendications 1 à 7 dans lequel le solvant organique utilisé pour extraire la couche alcaline dans l'étape (c) est choisi parmi le toluène, le chloroforme, le dichlorométhane et l'acétate d'éthyle.

9. Procédé tel que revendiqué dans la revendication 8
dans lequel le solvant organique est du chloroforme.

10. Procédé tel que revendiqué dans l'une quelconque des revendications 1 à 9 dans lequel l'acide minéral utilisé dans l'étape (d) pour neutraliser la solution alcaline est choisi parmi l'acide chlorhydrique et l'acide sulfurique.

11. Procédé tel que revendiqué dans la revendication 10 dans lequel l'acide minéral est de l'acide chlorhydrique.

12. Procédé tel que revendiqué dans l'une quelconque des revendications 1 à 11 dans lequel le solvant organique utilisé dans l'étape (d) pour extraire la solution neutralisée est choisi parmi l'acétate d'éthyle, le chloroforme et le dichlorométhane.

13. Procédé tel que revendiqué dans la revendication 12 dans lequel le solvant organique est de l'acétate d'éthyle.

14. Procédé tel que revendiqué dans l'une quelconque des revendications 1 à 13 dans lequel le solvant organique approprié ou les mélanges à partir d'un tel solvant utilisés dans l'étape (e) pour cristalliser le (-)secoisolaricirésinol sont choisis parmi l'acétone et les mélanges acétone-éther de pétrole, acétone-hexane, acétate d'éthyle-éther de pétrole et acétate d'éthyle-hexane.

15. Procédé tel que revendiqué dans la revendication 14 dans lequel le mélange de solvants est un mélange acétone-éther de pétrole.

16. Procédé tel que revendiqué dans l'une quelconque des revendications 1 à 15 dans lequel le solvant organique utilisé dans l'étape (f) pour dissoudre le (-)secoisolaricirésinol est choisi parmi l'acétonitrile, le méthanol, le tétrahydrofurane, le chloroforme et le dichlorométhane.

17. Procédé tel que revendiqué dans la revendication 16 dans lequel le solvant est de l'acétonitrile.

18. Procédé tel que revendiqué dans l'une quelconque des revendications 1 à 17 dans lequel l'halogénure de triphénylphosphine utilisé dans l'étape (f) est choisi parmi le bromure de triphénylphosphine et le chlorure de triphénylphosphine.

19. Procédé tel que revendiqué dans la revendication 18 dans lequel l'halogénure de triphénylphosphine est du bromure de triphénylphosphine.

20. Procédé tel que revendiqué dans l'une quelconque des revendications 1 à 19 dans lequel la température de réaction de l'étape (f) est de 60°C et la durée de la réaction dans l'étape (f) est de 2 heures.

21. Procédé tel que revendiqué dans l'une quelconque des revendications 1 à 20 dans lequel la colonne utilisée pour la chromatographie dans l'étape (g) pour purifier le (-) 3,4-divanillyle tétrahydrofurane est choisie parmi le gel de silice, le florosil, l'alumine et la célite.

22. Procédé tel que revendiqué dans la revendication 21 dans lequel la colonne utilisée pour la chromatographie comprend du gel de silice.
